# DEMANDE DE BREVET EUROPEEN

(11) **EP 0 890 349 A1**
(43) Date de publication de la demande: **13.01.1999**
(21) Numéro de dépôt: 98401724.4
(22) Date de dépôt: 08.07.1998
(51) Int. Cl.: A61F 5/455

(54) **Dispositif hygiénique pour incontinence urinaire d'une personne de sexe féminin**

(30) Priorité: 09.07.1997 FR 9708710
(71) Demandeur: Popescu, Aurel, 92350 Villemomble (FR); Denoyer, Colette, 92350 Villemomble (FR)
(72) Inventeur: Popescu, Aurel, 92350 Villemomble (FR); Denoyer, Colette, 92350 Villemomble (FR)
(74) Mandataire: Lejet, Christian

(57) **Abrégé**

La présente invention concerne un dispositif hygiénique pour incontinence urinaire d'une personne de sexe féminin, le dispositif comportant une partie de positionnement (16) destinée à être introduite dans l'entrée du vagin de la patiente, une partie de déflexion (10) du flux urinaire destinée à faire face au méat urétral de la patiente, et une partie de collecte (22) du flux urinaire destinée à être raccordée par un conduit (24) à une poche collectrice (26).

La partie de déflexion (10) est réalisée en un matériau élastique souple et elle délimite avec le pubis de la patiente un volume (20) de régulation du flux d'urine en amont de la poche collectrice (26).

Selon l'invention, le dispositif hygiénique comprend, en outre, une partie de positionnement (16) destinée à être introduite dans l'entrée du vagin de la patiente.

## Description

La présente invention concerne de manière générale les dispositifs destinés à être maintenus sur le corps de personnes de sexe féminin souffrant d'incontinence urinaire, passagère ou chronique, et permettant de collecter l'urine émise par ces malades dans une poche collectrice raccordée à ce dispositif.

De tels dispositifs sont déjà connus par de nombreux documents. Par exemple, le document FR-A-2 532 837 divulgue un dispositif comprenant un godet péri-urétral, un coussin extérieur et un soufflet tubulaire élastique qui s'étend entre les deux premiers éléments ainsi que des moyens appropriés pour maintenir le dispositif en place et pour collecter l'urine qui s'écoule à travers ce dispositif. Le godet péri-urétral est moulé d'une seule pièce en une matière molle et compressible et a des parois de forte épaisseur qui forment des surfaces arrondies douces appropriées pour s'appuyer de manière étanche contre les surfaces du plancher péri-urétral et de l'entrée du vagin. La paroi du godet se prolonge en une protubérance élastique déflectrice d'urine qui se loge dans l'entrée du vagin. Un conduit guide l'urine vers un collecteur. Un orifice muni d'un clapet laisse l'air pénétrer dans le dispositif afin d'éviter l'établissement d'une dépression à l'intérieur de ce dernier.

Le document FR-B-2 602 136 divulgue un dispositif comprenant une cuvette péri-urétrale réalisée en un matériau élastomère souple et présentant une cavité réceptrice d'urine s'ouvrant vers le haut et un rebord continu s'étendant autour de cette cavité. La cuvette comprend également une partie formant col qui s'étend, en étant venue de matière avec elle, vers le bas et présente un passage communiquant avec la cavité. La partie formant col est agencée de façon à être raccordée à un tube de drainage, et, d'autre part, un coussin adhésif élastique fixé extérieurement sur la partie formant col. Ce coussin comporte une paire de sections latérales offrant des surfaces adhésives plus larges faisant face latéralement qui sont situées le long de côtés opposés de la partie formant col en vue de venir au contact, de manière adhésive, avec les surfaces latérales des petites lèvres de la malade qui font face vers l'intérieur.

Le document EP-A-0 348 071 divulgue un dispositif comportant une coupelle péri-urétrale, un coussin externe en matériau doux, souple et résilient, et un soufflet tubulaire élastique compressible s'étendant entre une ouverture inférieure de la coupelle et l'entrée d'un passage de drainage de fluide s'étendant à travers le coussin. Le passage de drainage s'étend vers l'avant selon une direction générale horizontale le long du plan moyen sagittal du coussin, et la surface inférieure du coussin est pourvue de portions de surfaces disposées latéralement et de manière générale horizontalement, et, de préférence également, d'une portion de surface médiane coplanaire, pour répartir les forces et éviter l'inversion du coussin lorsque l'utilisatrice s'assied. Un tube de drainage flexible a une extrémité reliée de façon détachable au passage de drainage du coussin au moyen d'un couplage tubulaire ayant un insert reçu dans la sortie de ce passage. Le couplage comporte un évent et une valve dirigés latéralement, et une projection du couplage en forme de T (en section) s'étendant vers le haut est reçue à frottement dans une fente en forme de T du coussin pour empêcher la rotation du couplage par rapport au coussin et pour maintenir le couplage en alignement, et en communication fluidique, avec le passage de drainage du coussin.

Le document US-A-5 632 736 décrit un dispositif essentiellement prévu pour que l'utilisatrice puisse uriner debout pour éviter tout contact non-hygiénique avec un siège de toilette. Le problème posé ici est celui des fuites d'urine, d'où une abondance des barrières d'étanchéité. Ce document ne traite donc pas d'une incontinence (mictions fréquentes et incontrôlable par gouttes), mais d'une miction normale (jet sous pression), ce qui est très différent en termes de flux et de pression.

Le document US-A-4 233 978 décrit un dispositif hygiénique pour incontinence urinaire d'une personne de sexe féminin, le dispositif comportant une partie de déflexion du flux urinaire destinée à faire face au méat urétral de la patiente, et une partie de collecte du flux urinaire destinée à être raccordée par un conduit à une poche collectrice. La partie de déflexion est réalisée en un matériau élastique souple et elle délimite avec le pubis de la patiente un volume de régulation du flux d'urine en amont de la poche collectrice.

Ce document correspond au préambule de la première revendication.

Ces dispositifs connus présentent tous l'inconvénient d'être relativement complexes, et donc relativement onéreux à fabriquer. De plus, leur utilisation par la patiente n'est pas aisée.

La présente invention se place dans ce contexte et a pour objet de proposer un dispositif hygiénique pour collecter l'urine de patientes souffrant d'incontinence urinaire, de structure assez simple pour pouvoir être réalisé à moindres coûts, qui soient simples à utiliser par la patiente elle-même, qui soient étanches, qui ne causent aucune douleur à la patiente, et même qui soient confortables pour elle.

Dans ce but, la présente invention a pour objet un dispositif hygiénique pour incontinence urinaire d'une personne de sexe féminin, tel que décrit dans le document précité US-A-4 233 978.

Selon l'invention, le dispositif comprend, en outre, une partie de positionnement destinée à être introduite dans l'entrée du vagin de la patiente.

Cette partie de positionnement affecte, de préférence, la forme d'une protubérance sensiblement cylindrique dont l'extrémité est en forme de calotte hémisphérique.

Selon une caractéristique de l'invention, le volume de régulation délimité par la partie de déflexion et par le pubis de la patiente est rendu étanche par rapport à l'extérieur par le bord continu curviligne de la partie de déflexion, le bord étant appliqué sur le pourtour du pubis de la patiente par l'intermédiaire d'un adhésif.

De façon préférentielle, l'adhésif est constitué d'une mousse auto-adhésive double-face.

Selon un mode de réalisation avantageux, la partie de déflexion est réalisée en un matériau élastomère.

Selon un autre mode de réalisation, la partie de déflexion est réalisée dans un matériau thermoplastique de grade médical.

Selon encore un autre mode de réalisation, la partie de déflexion est réalisée en un latex alimentaire.

Dans tous ces modes de réalisation, il sera avantageux de prévoir que la partie de positionnement, la partie de déflexion et la partie de collecte soient réalisées en une seule pièce.

D'autres buts, caractéristiques et avantages de la présente invention ressortiront plus clairement de la description qui suit d'un exemple de réalisation donné à titre illustratif, en référence aux dessins annexés sur lesquels:

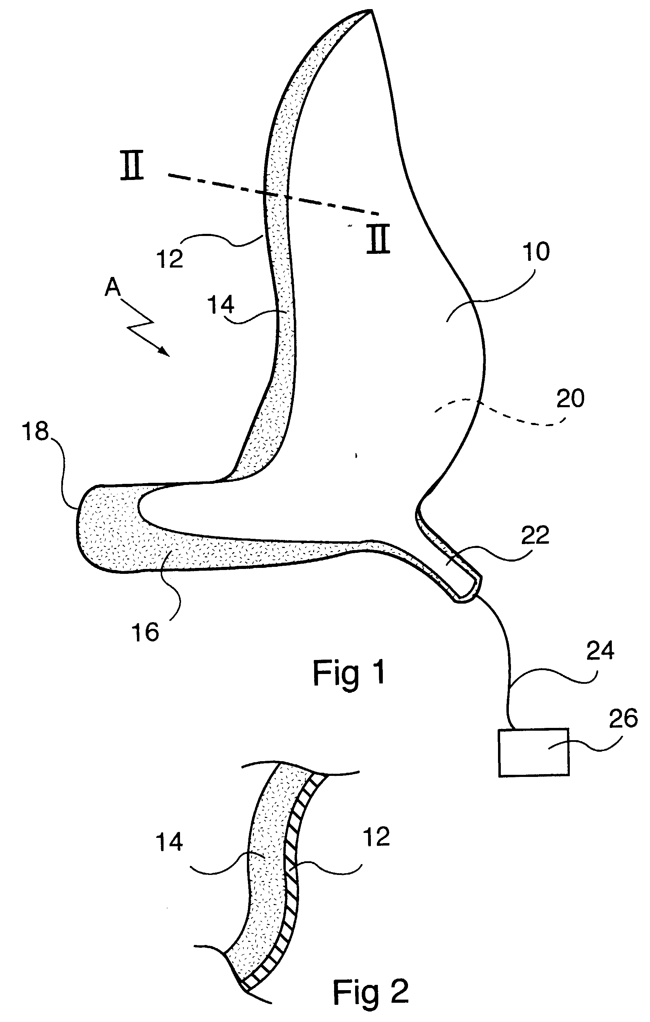

- La Figure 1 représente une vue de côté, en coupe partielle, du dispositif selon la présente invention;
- La Figure 2 représente une vue en coupe selon la ligne II-II de la Figure 1, et

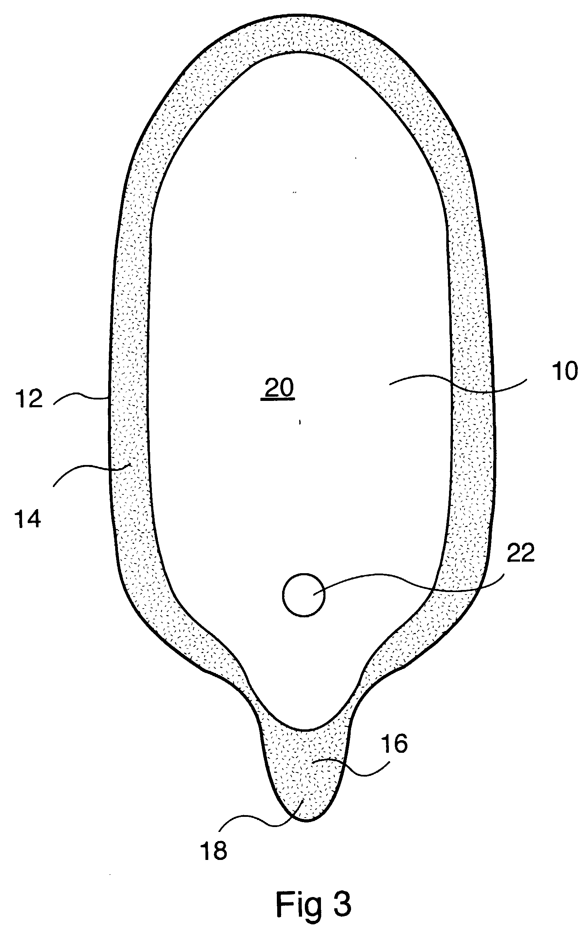

- La Figure 3 représente une vue de face du dispositif selon la présente invention, dans la direction de la flèche A de la Figure 1.

En référence aux Figures, on a représenté un dispositif hygiénique pour incontinence urinaire d'une personne de sexe féminin, réalisé selon la présente invention. Ce dispositif est destiné à être maintenu sur le corps de la patiente pour collecter les urines émises par elle, et il est destiné à être raccordé à une poche collectrice de ces urines.

De façon plus précise, le dispositif selon la présente invention comporte principalement un déflecteur 10, destiné à être placé devant le méat urétral de la patiente, et il est en forme de coquille creuse. Le déflecteur 10 est pourvu d'un bord continu curviligne 12 relativement rigide et destiné à être appliqué sur le pourtour du pubis de la patiente. Pour ce faire, le bord 12 est muni d'une bande de mousse auto-adhésive 14, d'environ 1 centimètre de large, de préférence double-face et amovible.

Le maintien en place du dispositif sur la patiente est assuré de plus par une partie de positionnement 16, sous forme de protubérance sensiblement cylindrique, d'environ 2 centimètres de diamètre, et dont l'extrémité 18 est en forme de calotte hémisphérique. La protubérance 16 a une longueur d'environ 3 centimètres, et elle est destinée à être introduite dans l'entrée du vagin de la patiente.

De cette manière, on est assuré que le dispositif hygiénique selon la présente invention est parfaitement maintenu en place, à l'aide de la protubérance 16 et du bord adhésif 14, ce dernier assurant, en outre, l'étanchéité du volume intérieur 20, délimité d'une part par le dispositif et d'autre part par le pubis de la patiente, par rapport à l'extérieur.

Le dispositif comporte également une canule 22, formant un raccord pour la fixation, de préférence amovible, d'un conduit souple 24, reliant le volume intérieur 20 à une poche collectrice, symbolisée en 26. La canule 22 est, de préférence, d'une seule pièce avec le déflecteur 10.

Selon la présente invention, le déflecteur 10 est réalisé en un matériau élastique souple, tel qu'un élastomère ou un matériau thermoplastique de grade médical, par exemple un latex alimentaire. De cette façon, le déflecteur forme un soufflet qui joue un double rôle. Il joue d'abord le rôle de déflecteur souple du jet d'urine sortant du méat urétral, ce qui contribue à éviter la génération de bruits désagréables.

Il joue ensuite un rôle de régulation du flux d'urine en amont de la poche collectrice 26. En effet, le déflecteur 10 étant souple, il délimite avec le pubis de la patiente un volume 20 variable. Si le flux urinaire en provenance de l'urètre est supérieur au flux maximal susceptible de traverser la canule 22, alors le volume 20 peut augmenter pour absorber temporairement cet excès de flux arrivant par rapport au flux sortant et, de la sorte, éviter que ce flux en excès ne sorte du volume 20 par un autre endroit que la canule 22. Un tel résultat est particulièrement important à obtenir dans le cas où la canule 22, et/ou le conduit souple 24, est bouché ou obstrué, par suite par exemple d'un mouvement ou d'un changement de position de la patiente.

D'autre part, la canule 22 étant raccordée au déflecteur souple 10 et au conduit 24, la canule 22 peut être orientée dans un grand nombre d'orientations par rapport au déflecteur 10, selon les circonstances, de sorte que le conduit 24 peut adopter un grand nombre de positions, la patiente pouvant choisir celle qui lui procure le moins de gêne possible.

On a donc bien réalisé selon la présente invention un dispositif hygiénique pour collecter l'urine de patientes souffrant d'incontinence urinaire, dont la structure est très simple, de sorte que ce dispositif hygiénique peut être réalisé à moindres coûts. Un tel dispositif est particulièrement simple à utiliser par la patiente elle-même, sa mise en position étant des plus faciles. Ce dispositif est parfaitement étanche, grâce au bord adhésif et à l'élasticité du déflecteur procurant un volume de stockage temporaire de l'urine en amont de la poche collectrice. Ce dispositif étant réalisé en un matériau élastique souple, tel qu'un élastomère ou un matériau thermoplastique de grade médical, par exemple un latex alimentaire, n'est pas susceptible de causer une douleur à la patiente, et peut même être relativement confortable pour elle.

Bien entendu, l'invention n'est pas limitée au mode de réalisation qui a été décrit, mais elle est susceptible au contraire de recevoir de nombreuses modifications qui apparaîtront à l'homme du métier. En particulier, la forme et les dimensions du dispositif hygiénique selon la présente invention pourront être adaptées aux dimensions ou à la morphologie des différentes patientes à traiter.

## Revendications

1. Dispositif hygiénique pour incontinence urinaire d'une personne de sexe féminin, le dispositif comportant une partie de déflexion (10) du flux urinaire destinée à faire face au méat urétral de la patiente, et une partie de collecte (22) du flux urinaire destinée à être raccordée par un conduit (24) à une poche collectrice (26), la dite partie de déflexion (10) étant réalisée en un matériau élastique souple et délimitant avec le pubis de la patiente un volume (20) de régulation du flux d'urine en amont de la poche collectrice (26), caractérisé en ce qu'il comprend, en outre, une partie de positionnement (16) destinée à être introduite dans l'entrée du vagin de la patiente.

2. Dispositif selon la revendication 1, caractérisé en ce que la partie de positionnement (16) affecte la forme d'une protubérance sensiblement cylindrique dont l'extrémité (18) est en forme de calotte hémisphérique.

3. Dispositif selon la revendication 2, caractérisé en ce que la dite protubérance (16) a une longueur de 3 cm environ et un diamètre de 2 cm environ.

4. Dispositif selon l'une quelconque des revendications précédentes, caractérisé en ce que le volume de régulation (20) délimité par la partie de déflexion (10) et par le pubis de la patiente est rendu étanche par rapport à l'extérieur par le bord continu curviligne (12) de la partie de déflexion (10), le dit bord (12) étant appliqué sur le pourtour du pubis de la patiente par l'intermédiaire d'un adhésif (14) constitué d'une mousse auto-adhésive double-face.

5. Dispositif selon l'une quelconque des revendications précédentes, caractérisé en ce que la partie de déflexion (10) est réalisée en un matériau élastomère.

6. Dispositif selon l'une quelconque des revendications 1 à 4, caractérisé en ce que la partie de déflexion (10) est réalisée dans un matériau thermoplastique de grade médical.

7. Dispositif selon l'une quelconque des revendications 1 à 4, caractérisé en ce que la partie de déflexion (10) est réalisée en un latex alimentaire.

8. Dispositif selon l'une quelconque des revendications précédentes, caractérisé en ce que la partie de positionnement (16), la partie de déflexion (10) et la partie de collecte (22) sont réalisées en une seule pièce.
